# EUROPEAN PATENT SPECIFICATION

(11) **EP 0 824 024 B1**
(45) Date of publication and mention of the grant of the patent: **20.08.2003**
(21) Application number: 97109876.9
(22) Date of filing: 17.06.1997
(51) Int. Cl.: A61M 16/12

(54) **Method for mixing gases and a device for mixing gases**
Verfahren und Vorrichtung zur Mischung von Gasen
Méthode et dispositif pour mélanger des gaz

(30) Priority: 12.08.1996 SE 9602959
(43) Date of publication of application: 18.02.1998
(73) Proprietor: Siemens-Elema AB, 171 95 Solna 1 (SE)
(72) Inventor: Rydgren, Göran, 230 44 Bunkeflostrand (SE); Lindberg, Lars, 246 57 Barsebäck (SE)

(56) References cited:
- EP-A- 0 640 357
- EP-A- 0 659 445
- WO-A-96/11718

## Description

The present invention relates to a method according to the preamble to claim 1.

The present invention also relates to a device according to the preamble to claim 4.

In recent years, the gas NO has attracted widespread interest in different kinds of therapy. A plurality of positive effects has been found when small amounts of NO are administered to a patient via the airways. For example, NO has a relaxing effect on smooth muscle, thereby improving oxygenation and reducing blood pressure across the lung in patients with severe pulmonary disease. More extensive descriptions of the effects conveyed by NO are provided in e.g. WO 92/10228 and US-A-5,427,797.

NO, usually diluted with N₂, is supplied in gas cylinders and subsequently mixed with a respiratory gas, usually a mixture of air and oxygen (O₂), before the final mixture is delivered to the patient. Examples of prior art systems are described in EP -A-0 659 445 and SE-C-502 724.

The biggest problem with NO is that it is a highly reactive gas and forms, with O₂, NO₂ - a highly toxic gas even in small concentrations. Since respiratory gas often contains an elevated concentration of O₂, typically 50-80% O₂, it will be realized that special measures may be necessary to minimize the amount of NO₂ delivered to the patient.

The gas containing NO can be supplied as close as possible to the patient, even inside the patient. However, this conveys the disadvantage that the two gases may not have time to mix thoroughly, and a heterogeneous gas mixture could be carried down into the lungs.

Another option is to mix the two gases continuously as they flow at a constant rate past an inspiratory line, and the patient would then draw a fresh mixture into her/his lungs at every breath. However, implementing this option is difficult when the patient is incapable of spontaneous breathing with an adequate volume. Moreover, large amounts of gas would be consumed, and gas containing NO would have to be evacuated to prevent a rise in the level of NO₂ in the room.

Another possibility is to mix the gases in the customary fashion and install an NO₂ absorber or an NO₂ filter before the patient. However, a disadvantage here is the difficulty in determining the supplied concentration of NO. An absorber must be monitored to keep it from becoming saturated, thereby losing its ability to absorb NO₂, and a filter must be arranged to keep NO₂ from escaping into the room.

Until now, the conversion of NO into NO₂ was believed to occur in proportion to the squared concentration of NO times the level of O₂ times time (k₂*(NO)²*O₂*t). In addition, a small amount of NO₂ may be present in the O₂-NO mixture. This amount is proportional to the concentration of NO (k₀*NO).

However, recently conducted experiments have disclosed an additional factor. NO was then found to have a property which causes initial formation of NO₂, at the instant of mixture with oxygen, which is proportional only to the square of the NO concentration and the O₂ concentration (k₁*(NO)²*O₂).

So the final conversion equation, designating the concentration of NO₂, is as follows:

NO₂ = k₀ * NO + k₁ * (NO)² * O₂ + k₂ * (NO)² * O₂ * t

One objective of the present invention is to utilize this recently discovered phenomenon in the formation of NO₂ for achieving a method for minimizing the formation of NO₂ when two gases containing NO and O₂ respectively are mixed.

Another objective of the invention is to achieve a device which is designed to minimize the formation of NO₂ when two gases containing NO and O₂ respectively are mixed.

One such method is achieved in accordance with the invention by the specific features set forth in the characterizing part of claim 1.

Advantageous improvements of the method according to the invention are apparent from the dependent claims of claim 1.

When gas containing NO is added to gas containing O₂ at a plurality of mixing points, the contribution to the formation of NO₂, according to the newly discovered property, can be reduced. Assume e.g. that the first gas contains 1000 ppm of NO in N₂. When it is diluted to form the second gas with a concentration of 100 ppm, k₁*100² ppm of NO₂ are initially formed. If two mixing points are employed, the initial formation (at each point) becomes k₁*50² ppm of NO₂. Total initial formation then becomes 2*(K₁*50²) ppm, i.e. a reduction in initial NO₂ by a factor of 2.

A device is achieved in accordance with the invention in that the device according to the preamble encompasses the distinctive features set forth in the characterizing part of claim 4.

Advantageous improvements of the device according to the invention are set forth in the dependent claims of claim 4.

One embodiment of the device and method according to the invention will be described below in greater detail, referring to the figures in which
FIG. 1 shows the device connected to a ventilator for dosing NO to a patient and
FIG. 2 shows the device itself in greater detail.

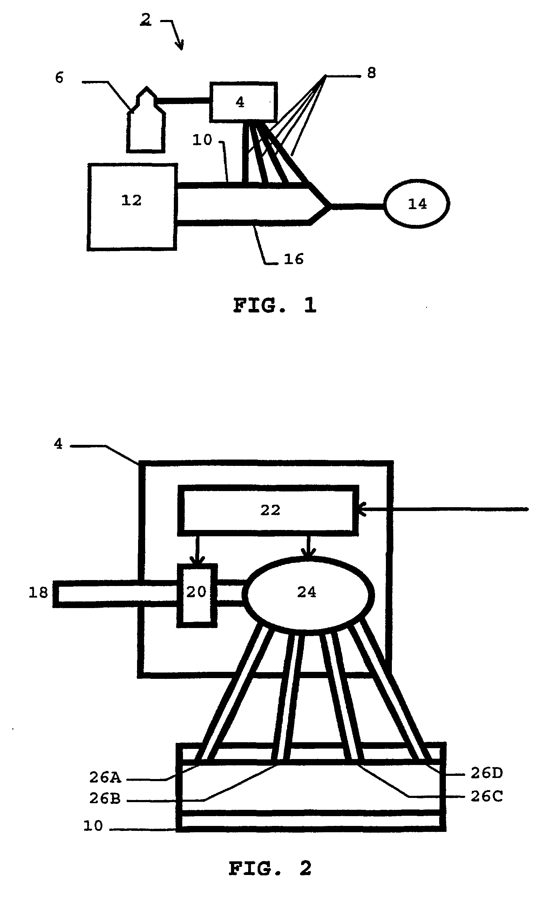

A system 2 for dosing NO to a patient 14 is shown in FIG. 1. The system 2 comprises a device 4 for dosing a gas containing NO from a gas source 6 to an inspiratory line 10 via dosing lines 8.

The inspiratory line 10 is connected to a ventilator 12 and carries a respiratory gas from same to the patient 14. The ventilator 12 can be an existing ventilator on the market such as the Servo Ventilator 300, Siemens-Elema, Solna, Sweden.

Air expired by the patient 14 is carried back to the ventilator 12 in an expiratory line 16.

As FIG. 2 shows, the device 4 has a gas connector 18, which is coupled to the gas source 6, and a flow regulator 20 for regulating the flow required to achieve a pre-defined concentration of NO in the final mixture supplied to the patient 14. The flow regulator 20 is controlled by a control device 22. As indicated by an arrow in the figure, the control device 22 receives information on the flow of respiratory gas in the inspiratory line 10 and then controls the flow regulator 20 according to the received flow value and a reference value for the pre-defined concentration. In the known manner, the flow of respiratory gas can be obtained from the ventilator 12 or a separate flow meter (not shown in the figures).

The dosed flow is carried to a flow divider 24 which distributes this flow via a first mixing point 26A, a second mixing point 26B, a third mixing point 26C and a fourth mixing point 26D, to the inspiratory line 10. The distance between the mixing points 26A-26D is selected so a homogenous gas mixture is achieved before each new mixing point 26B-26D in the direction of respiratory gas flow. The number of mixing points can vary, but two to ten mixing points are appropriate. Too many mixing points 26A-26D could result in needlessly long mixing distances.

Since NO reacts with O₂ to form NO₂ according to the equation

NO₂ = k₀ * NO + k₁ * (NO)² * O₂ + k₂ * (NO)² * O₂ * t

in which k₀ - k₂ are constants, and the first term designates the concentration of NO₂ in the gas source, the second term designates the initial formation of NO₂ in the mixture of gases and the third term designates the time-related formation of NO₂ when the gases have been mixed, it will be realized that subdivision into a plurality of mixing points reduces the second term, i.e. the initial formation of NO₂.

Assume that NO is to be supplied to an infant, e.g. a premature neonate with respiratory problems, through an 1.5 m long inspiratory tube. A typical flow would then be one l/min with an oxygen content of 90% and administration of 100 ppm of NO.

With four mixing points, as in FIG. 2, 10 cm apart and the same dosing flow at each mixing point, a concentration of NO of 25 ppm results after the first mixing point 26A, 50 ppm after the second mixing point 26B, 75 ppm after the third mixing point 26C and 100 ppm after the fourth mixing point 26D. When the first mixing point is 1.5 m from the patient (and the others are at 1.4, 1.3 and 1.2 m), 0.43, 0.42, 0.40 and 0.39 ppm of NO₂ are formed at the respective mixing point. This amounts to a total of 1.63 ppm.

The introduction of 100 ppm of NO at one mixing point at 1.2 m, i.e. the shortest distance, would have resulted in the formation of 4.69 ppm of NO₂. In view of the toxicity of NO₂, the difference is particularly significant.

Introduction does not have to be into the inspiratory tube but can be made in a special mixing chamber or the like. The most important thing is for mixing to occur at a plurality of mixing points and for homogenous mixing to take place before each new mixing point for the very best results to be achieved.

The distance between the mixing points 26A-26D does not need to be constant but can vary.

## Claims

1. A method for mixing flowing gases, said method entailing the mixture of a first gas, containing NO, with a second gas, containing O₂, so a predetermined concentration of NO is achieved with minimized formation of NO₂ in the final gas mixture, **characterized in that** the first gas is added to the second gas at at least two, or preferably more, mixing points arranged at specific distances from each other.

2. A method according to claim 1, **characterized in that** the first gas is added at the same flow rate at each mixing point.

3. A method according to claim 1 or 2, **characterized in that** the specific distance is selected so homogenous mixing of the first gas and the second gas is achieved before each new mixing point in the direction of gas flow.

4. A device (4), intended for connection to a ventilator system (12) for the controlled addition of NO to a respiratory gas containing O₂, the device (4) comprising a gas source (6) containing NO, **characterized in that** the device (4) is devised to introduce the gas containing NO into the respiratory gas at at least two, or preferably more, mixing points (26A-26D), arranged at specific distances from each other, so the gases become intermixed.

5. A device according to claim 4, **characterized in that** the device (4) contains a flow regulator (20) for regulating the total flow of the gas containing NO from the gas source (6) and a flow divider (24) for distributing the total flow of the gas containing NO to the mixing points (26A-26D).

6. A device according to claim 5, **characterized in that** the flow divider (24) is adapted to distribute the total flow of the gas containing NO so an identical flow of the gas containing NO is supplied to each mixing point (26A-26D).

7. A device according to claim 5 or 6, **characterized in that** the device is adapted to be connected to an inspiratory line of the ventilator system so the respiratory gas flows, at least periodically, past the mixing points (26A-26D), and the flow regulator (20) is devised to regulate the total flow of the gas containing NO according to the flow of the respiratory gas.

## Patentansprüche

1. Ein Verfahren zum Mischen strömender Gase, wobei das Verfahren das Mischen eines ersten, NO enthaltenen Gases mit einem zweiten, O₂ enthaltenden Gases umfasst, so dass eine vorbestimmte Konzentration von NO unter minimaler Bildung von NO₂ in der endgültigen Gasmischung erzielt wird, **dadurch gekennzeichnet, dass** das erste Gas dem zweiten Gas an zumindest zwei und vorteilhafterweise mehreren Mischpunkten zugeführt wird, die in spezifischen Abständen voneinander angeordnet sind.

2. Ein Verfahren nach Anspruch 1, **dadurch gekennzeichnet, dass** das erste Gas mit der gleichen Durchflussgeschwindigkeit in jedem Mischpunkt zugeführt wird.

3. Ein Verfahren nach Anspruch 1 oder 2, **dadurch gekennzeichnet, dass** der spezifische Abstand so gewählt wird, dass ein homogenes Mischen des ersten Gases und des zweiten Gases vor jedem neuen Mischpunkt in der Richtung des Gasflusses erzielt wird.

4. Eine Vorrichtung (4), vorgesehen für die Verbindung mit einem Ventilatorsystem (12) zum gesteuerten Zusatz von NO zu einem O₂ enthaltenden Atemgas, wobei die Vorrichtung eine NO enthaltende Gasquell (6) aufweist, **dadurch gekennzeichnet, dass** die Vorrichtung (4) ausgebildet ist, das NO enthaltende Gas an zumindest zwei oder vorzugsweise mehreren Mischpunkten (26A-26D), die in spezifischen Abständen voneinander angeordnet sind, zuzuführen, so dass die Gase vermischt werden.

5. Eine Vorrichtung nach Anspruch 4, **dadurch gekennzeichnet, dass** die Vorrichtung (4) einen Flussregler (20) zum Regeln des Gesamtflusses des NO enthaltenden Gases von der Gasquelle (6) und einen Flussteiler (24) zum Verteilen des Gesamtflusses von NO enthaltenden Gases zu den Mischpunkten (26A-26D) aufweist.

6. Eine Vorrichtung nach Anspruch 5, **dadurch gekennzeichnet, dass** der Flussteiler (24) angepasst ist, den Gesamtfluss von NO enthaltendem Gas so zu verteilen, dass ein identischer Gasfluss von NO enthaltendem Gas jedem Mischpunkt (26A-26D) zugeführt wird.

7. Eine Vorrichtung nach Anspruch 5 oder 6, **dadurch gekennzeichnet, dass** die Vorrichtung angepasst ist, an die Inspirationsleitung eines Ventilatorsystems angeschlossen zu werden, so dass der Atemgasfluss zumindest periodisch an den Mischpunkten (26A-26D) vorbeiströmt und der Flussregler (20) ausgebildet ist, den Gesamtfluss des NO enthaltenden Gases entsprechend dem Fluss des Atemgases zu regeln.

## Revendications

1. Procédé de mélange de gaz en écoulement, le procédé consistant à mélanger un premier gaz contenant du NO à un deuxième gaz contenant du O₂, de manière à obtenir une concentration déterminée à l'avance de NO avec une formation minimisée de NO₂ dans le mélange final de gaz, **caractérisé en ce qu'**on ajoute le premier gaz au deuxième gaz en au moins deux points de mélange, ou de préférence en plus de deux points de mélange, disposés à des distances précises l'un de l'autre.

2. Procédé selon la revendication 1,
**caractérisé en ce qu'**on ajoute le premier gaz au même débit en chaque point de mélange.

3. Procédé selon la revendication 1 ou 2,
**caractérisé en ce qu'**on choisit la distance précise de manière à obtenir un mélange homogène du premier gaz et du deuxième gaz avant chaque nouveau point de mélange dans le sens du courant de gaz.

4. Dispositif (4) destiné à être relié à un système (12) de ventilateur pour l'addition commandée de NO à un gaz respiratoire contenant du O₂, le dispositif (4) comprenant une source (6) de gaz contenant du NO,
**caractérisé en ce que** le dispositif (4) est conçu pour introduire le gaz contenant du NO dans le gaz respiratoire en au moins deux points (26A-26D) de mélange, ou de préférence en plus de deux points de mélange, disposés à des distances précises l'un de l'autre, de sorte que les gaz se mélangent.

5. Dispositif selon la revendication 4,
**caractérisé en ce que** le dispositif (4) comporte un régulateur (20) de débit pour réguler le débit total du gaz contenant du NO en provenance de la source (6) de gaz et un diviseur (24) de débit pour répartir le débit total du gaz contenant du NO entre les points (26A-26D) de mélange.

6. Dispositif selon la revendication 5,
**caractérisé en ce que** ce diviseur (24) de débit est conçu pour répartir le débit total du gaz contenant du NO de manière à obtenir un débit identique du gaz contenant du NO à chaque point (26A-26D) de mélange.

7. Dispositif selon la revendication 5 ou 6,
**caractérisé en ce que** le dispositif est conçu pour être relié à une ligne d'inspiration du système ventilateur de façon à ce que le gaz respiratoire passe, au moins par période, devant les points (26A-26D) de mélange, et le régulateur (20) de débit est conçu pour réguler le débit total du gaz contenant du NO suivant le débit du gaz respiratoire.
